# EUROPÄISCHE PATENTSCHRIFT

(11) **EP 3 786 150 B1**
(45) Veröffentlichungstag und Bekanntmachung des Hinweises auf die Patenterteilung: **30.07.2025**
(21) Anmeldenummer: 20000241.8
(22) Anmeldetag: 03.07.2020
(51) Int. Cl.: C07C 69/704, C11D 3/20

(54) **HYDROXYCARBONSÄUREESTER ENTHALTENDE WASCH- UND REINIGUNGSMITTEL UND VERWENDUNG VON HYDROXYCARBONSÄUREESTERN**
DETERGENTS AND CLEANING AGENTS CONTAINING HYDROXYCARBOXYLIC ACID ESTERS AND USE OF HYDROXYCARBOXYLIC ACID ESTERS
DÉTERGENTS ET PRODUITS D'ENTRETIEN CONTENANT DES ESTERS D'ACIDES HYDROXYCARBOXYLIQUES ET UTILISATION D'ESTERS D'ACIDES HYDROXYCARBOXYLIQUES

(30) Priorität: 24.08.2019 DE 102019005969
(43) Veröffentlichungstag der Anmeldung: 03.03.2021
(73) Patentinhaber: WeylChem Performance Products GmbH, 65203 Wiesbaden (DE)
(72) Erfinder: Morschhäuser, Roman, 55122 Mainz (DE); Kchirid, Said, 63869 Heigenbrücken (DE); Preuschen, Judith, 55270 Sörgenloch (DE); Kuhse, Bo, 65195 Wiesbaden (DE)
(74) Vertreter: Ackermann, Joachim

(56) Entgegenhaltungen:
- WO-A1-2019/158409
- WO-A2-2019/157350
- CN-A- 109 293 505
- DE-A1- 102009 002 097
- P.R. ; CARLIER ET AL: "Studies on the Mechanism of the Asymmetric Epoxidation: A Ligand Variation Approach", JOURNAL OF ORGANIC CHEMISTRY, 1 January 1989 (1989-01-01), pages 4016 - 4018, XP055757179, Retrieved from the Internet <URL:https://pubs.acs.org/doi/pdf/10.1021/jo00278a004> [retrieved on 20201207]
- TIMOTHY R. NEWHOUSE ET AL: "A Tetradentate Ligand for the Enantioselective Ti(IV)-Promoted Oxidation of Sulfides to Sulfoxides: Origin of Enantioselectivity", JOURNAL OF THE AMERICAN CHEMICAL SOCIETY, vol. 134, no. 42, 12 October 2012 (2012-10-12), US, pages 17354 - 17357, XP055757356, ISSN: 0002-7863, DOI: 10.1021/ja305991k
- KSENIA PUMPOR ET AL: "Hexafluoroacetone as a Protecting and Activating Reagent. Regioselective Esterification of Aspartic, Malic, and Thiomalic Acid", MONATSHEFTE F?R CHEMIE - CHEMICAL MONTHLY, vol. 135, no. 11, 23 August 2004 (2004-08-23), pages 1427 - 1443, XP055059263, ISSN: 0026-9247, DOI: 10.1007/s00706-004-0183-9

## Beschreibung

Die Erfindung betrifft neue Wasch- und Reinigungsmittel enthaltend Hydroxycarbonsäureester, die sich von aliphatischen Hydroxycarbonsäuren und Alkylenglykolen ableiten. Diese Verbindungen sind ausgezeichnete Komplexbildner und lassen sich auf unterschiedlichsten Gebieten einsetzen, beispielsweise in Wasch- und Reinigungsmitteln, bei der Erdölgewinnung oder zur Wasserenthärtung.

Polymere Hydroxycarbonsäureester, wie polymere Zitronensäureester, sind aus der Literatur bekannt und können durch eine Kondensationsreaktion der Hydroxycarbonsäure mit ein- oder mehrwertigen Alkoholen hergestellt werden. Die Veresterungsreaktion wird dabei durch Erhitzen der Ausgangskomponenten durchgeführt, wobei sich in der Regel Oligomere oder Polymere mit undefinierten Kettenlängen bilden. Dabei besteht zudem das Risiko der Zersetzung der Hydroxycarbonsäure während der Reaktion, so dass unkontrolliert Nebenprodukte gebildet werden, die ihrerseits mit den Komponenten des Reaktionsgemisches reagieren. So zersetzt sich beispielsweise Zitronensäure leicht an ihrem Schmelzpunkt unter Verlust einer COOH Gruppe und daher sind Polyester mit reproduzierbarer Kettenlänge schwer herzustellen.

Aus der WO 2001/000463 A2 ist ein Verfahren zur Herstellung von Estern aliphatischer Carbonsäuren bekannt. Bei dem ein Reaktionsgemisch aus aliphatischen Carbonsäuren und ein- oder mehrwertigten Alkoholen für eine sehr kurze Zeit durch Mikrowellenbestrahlung auf sehr hohe Temperaturen weit oberhalb von 100°C erhitzt werden. Das Verfahren liefert nahezu quantitative Ausbeuten und es entstehen praktisch keine Nebenprodukte. Als ein Beispiel für eine aliphatische Carbonsäure wird Zitronensäure genannt.

CN 109293505 beschreibt ein Verfahren zur Umsetzung von Zitronensaft und Salzsäure unter der Einwirkung von Mikrowellenstrahlung. In dieser Schrift wird behauptet, dass durch dieses Verfahren Ethylenglykolcitrat hergestellt werde. Es ist aber nicht offenbart, ob während der Umsetzung Ethylenglykol zugegen ist und wann und wie Ethylenglykol in das Reaktionsgemisch eingebracht wurde. Außerdem wird der angesäuerte Zitronensaft über eine Stunde der Mikrowellenstrahlung in einem geschlossenen Behälter ausgesetzt. Unter solchen Bedingungen ist von einer teilweisen Zersetzung bzw. Polymerisation der Reaktanten auszugehen.

Aus der WO 2019/158409 A1 ist ein Verfahren zur Herstellung grenzflächenaktiver Kondensate von Zitronensäure bekannt. Die dabei erhaltenen Produkte weisen mindestens ein hydrophobes Strukturelement auf, welches eine Kohlenwasserstoffgruppe mit mindestens acht Kohlenstoffatomen darstellt. Die Kondensate können als Tenside, Emulgatoren oder Verdicker in Reinigungs- oder Pflegemitteln eingesetzt werden.

Polymere Zitronensäuren sind bereits für eine Reihe von Anwendungen aus der Literatur bekannt.

Yuzeng Zao et al. beschreiben in Desalination, Vol. 392, S. 1-7 (2016) (https://www.sciencedirect.com/science/article/pii/S0011916416301813) die Inhibierung der Bildung von Calciumsulfatablagerungen durch den Einsatz von Poly(zitronensäure)-Derviaten. Die eingesetzten Produkte weisen einen hohen Polymerisationsgrad und eine Vielzahl von Zitronensäureeinheiten auf.

A.T. Naeini et al. offenbaren in Nanomedicine, 6(4), S. 556-562 (2010) Copolymere aus Poly(zitronensäure- und Poly(ethylenglykol)-Blöcken als biokompatile Hybridmaterialien für die Nanomedizin.

N. Memarizadh et al. beschreiben in Environmental Science: Processes & Impacts, 2014, 16, 2380-2389 supramolekulare Systeme aus linear dendritischen Copolymeren und Indoxacarb als biologisch abbaubare und effiziente Nanopestizide. Als Copolymere werden dendritische Block-Copolymere aus Polyethylenglykol und Polyzitronensäure eingesetzt.

In Biomaterials 31(34), S. 9092-2108 (2010) beschreiben D. Gyawali et al. in situ vernetzbare biologisch abbaubare Polymere für die Erzeugung von Zellkulturen. Die vorgeschlagenen Polymere leiten sich von Polyethylenglykol, Maleinsäure und Zitronensäure ab und neigen zur Ausbildung von Hydrogelen.

In J. Polym. Environ (2012), 20: 291-298 beschreiben B. Tisserat et al. analytische Methoden zur Charakterisierung von Schäumen aus Poly(glycerincitrat), die durch Einwirkung von Mikrowellenstrahlung erzeugt worden sind. Bei der Herstellung der Polymeren werden äquimolare Mengen an Zitronensäure und Glycerin eingesetzt, so dass Polymere mit hohen Molekulargewichten resultieren.

In J. of Applied Polymer Science, Vol. 125, 3429-3437 (2012) beschreiben B. Tisserat et al. die Synthese von Polyestern, die sich von Zitronensäure und Glycerin ableiten. Zur Herstellung der Polyester werden die Ausgangsmaterialien in unterschiedlichen Mengenverhältnissen vorgelegt und anschließend erhitzt, wobei verschiedene Heizmethoden, darunter auch die Einwirkung von Mikrowellenstrahlung, angewendet werden. In Abhängigkeit von der verwendeten Heizmethode und den Mengenverhältnissen der Ausgangsmaterialien entstehen dabei unterschiedliche Produkte, die als Schaum, Gel oder als Flüssigkeit anfallen.

WO 92/16493 A1 beschreibt Zitronensäureester von Polyhydroxyverbindungen mit mindestens drei Hydroxygruppen, beispielsweise Polyglycerin oder Zuckeralkohole, und deren Verwendung in Wasch- und Reinigungsmitteln. Die Verbindungen können die Wirkung von anderen Waschmittelzusätzen verstärken.

Aus der DE 1,617,122 A sind wasserlösliche Salze von freie Carboxylgruppen enthaltenden Polyestern bekannt, deren Säurekomponenten aus einem Tri- oder Tetracarbonsäurerest besteht und deren Alkoholkomponenten sich von Verbindungen mit zwei aliphatischen Hydroxylgruppen ableiten. Beschrieben werden beispielsweise Polyester, die sich von Zitronensäure und Ethylenglykol ableiten. Dabei handelt es sich um hochmolekulare Harze, die sich in alkalischen Waschlaugen lösen. Diese Mittel erleichtern den Waschvorgang und erhöhen den Weißgrad der Wäsche.

Aus dem Stand der Technik sind folglich Ester von aliphatischen Hydroxycarbonsäuren und verschiedenen Alkoholen, beispielsweise Polyester abgeleitet von Zitronensäure und Glycerin oder anderen Polyolen, bekannt. Es wurden bereits Moleküle mit unterschiedlichen Kettenlängen beschrieben, die z.B. von zwei Zitronensäureeinheiten bis über einhundert Säureeinheiten umfassen können.

Niedermolekulare Ester von aliphatischen Hydroxycarbonsäuren mit aliphatischen Diolen wurden beispielsweise in den folgenden Dokumenten beschrieben.

Carlier et al. berichten in J. Org. Chem. 1989, 54, 4016-8 über eine Studie zum Mechanismus der asymmetrischen Epoxidierung. Dabei kommen Diglykolester der Weinsäure zum Einsatz.

Newhouse et al. berichten in JACS, (2012) 134, S. 17354-57 über vierzähnige Liganden für die enantioselektive Ti-(IV)-katalysierte Oxidation von Sulfiden zu Sulfoxiden. Dabei kommen unter anderem Diglykolester der Weinsäure als Liganden zum Einsatz.

Pumpor et al. beschreiben in Monatshefte für Chemie, 135, S. 1427-43 (2004) den Einsatz von Hexafluoraceton als Schutzgruppe. In diesem Artikel wird unter anderem ein Diester von Ethylenglykol und Äpfelsäure offenbart.

Darüber hinaus offenbaren die WO 2019/157350 A2 und die DE 10 2009 002 097 A1 verschiedene Ester von Hydroxycarbonsäuren, wie Zitronensäure, mit unterschiedlichen Glykolen.

Zur Herstellung von Estern aliphatischer Hydroxycarbonsäuren wurden Kondensationsreaktionen in verschiedenen Lösungsmitteln durchgeführt, beispielsweise in Wasser, Glycerin, Propylenglykol und Ethylenglykol. Dabei wurde überraschenderweise gefunden, dass Ester von Hydroxycarbonsäuren mit Alkylenglykolen oder mit Polyalkylenglykolen niedrigen Kondensationsgrades und hergestellt durch eine Beaufschlagung mit einem hohen Temperaturpuls sich ausgezeichnet als Komplexbildner für unterschiedliche Kationen eignen. So zeigt ein von Ethylenglykol und Zitronensäure abgeleiteter Ester, der insgesamt wenige Zitronensäureeinheiten aufweist, eine ausgezeichnete Tendenz zur Komplexbildung während ein von Glycerin und Zitronensäure abgeleiteter Ester, der ebenfalls insgesamt wenige Zitronensäureeinheiten aufweist, keine Tendenz zur Komplexbildung aufweist.

Aufgabe der vorliegenden Erfindung ist die Bereitstellung von chemischen Verbindungen, die sich von einfach zugänglichen Ausgangsmaterialien, vorzugsweise von Ausgangsmaterialien biologischen Ursprungs ableiten, die eine ausgezeichnete Komplexbildungstendenz aufweisen und die sich vorzugsweise in Wasch- und Reinigungsmitteln verwenden lassen.

Gegenstand der vorliegenden Erfindung sind Wasch- und Reinigungsmittel enthaltend Verbindungen der Formel (I)

(R¹OOC)ₐ-R²(OH)_{c}-COO-(CₙH₂ₙ-O)ₘ-OC-R³(OH)_{d}(COOR⁴)_{b} (I)

worin R¹ und R⁴ unabhängig voneinander Wasserstoff, ein Kation eines Metalls, ein Ammoniumkation, C₁-C₆-Alkyl, Cycloalkyl mit drei bis neun Ringkohlenstoffatomen, Aryl mit fünf bis zehn Ringkohlenstoffatomen, Aryl, das mit ein oder zwei Alkylgruppen substituiert ist, Aryl, das über eine Alkylengruppe mit der Carboxylgruppe verbunden ist, -(CₙH₂ₙ-O)ₘ-H oder -O-R²(COOR¹)ₐ₊₁ bedeuten,
R² und R³ unabhängig voneinander aliphatische Kohlenwasserstoffreste mit ein bis acht, vorzugsweise von zwei bis vier Kohlenstoffatomen sind,
a und b unabhänigig voneinander ganze Zahlen von 1 bis 4, vorzugsweise 1 bis 3 sind,
c eine ganze Zahl von 0 bis 4, vorzugsweise 1 bis 4, insbesondere 1 oder 2 und ganz besonders bevorzugt 1 ist,
d eine ganze Zahl von 1 bis 4, vorzugsweise 1 oder 2 und ganz besonders bevorzugt 1 ist,
n 2, 3 oder 4, vorzugsweise 2 oder 3 und insbesondere 2 bedeutet, und m 1, 2, 3 oder 4 ist, mit der Maßgabe, dass R¹ und R⁴ innerhalb eines Moleküls im Rahmen der gegebenen Definitionen unterschiedlich sein können.

Die Verbindungen der Formel (I) lassen sich auch in Form der Formel (la) darstellen 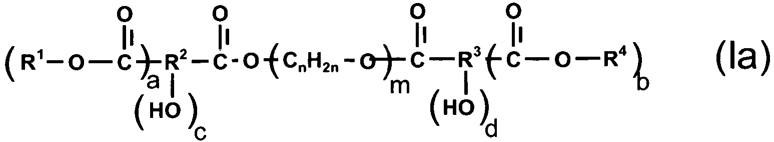 worin R¹, R², R³, R⁴, a, b, c, d, m und n die oben definierte Bedeutung besitzen. Durch NMR-Analyse kann der Schluss gezogen werden, dass sich bei der durch impulsförmige Temperaturerhöhung, beispielsweise durch Mikrowellenstrahlung vermittelten Kondensationsreaktion Moleküle mit wenigen Hydroxycarbonsäureeinheiten, beispielsweise mit 2 oder 3 Zitronensäureeinheiten, ausbilden. Die Hydroxycarbonsäureeinheiten, beispielsweise die Zitronensäureeinheiten, werden über Esterbindungen an Einheiten gebunden, die sich von Alkylenglykolen oder Di- bis Trialkylenglykolen als eingesetztem Lösungsmittel ableiten. Dabei entstehen niedermolekulare Verbindungen und keine Polymere.

Hydroxycarbonsäuren zeigten bei der Umsetzung mit anderen Alkoholen als (Poly)alkylenglykolen, beispielsweise bei der Umsetzung mit Glycerin eine sehr hohe Lösungsviskosität oder eine Braunfärbung und darüber hinaus zeigen die resultierenden Produkte keine oder nur eine geringe Komplexbildungstendenz.

Polyester enthaltend eine größere Anzahl an Hydroxycarbonsäureeinheiten und an Alkoholeinheiten weisen häufig eine zu hohe Viskosität auf und sind daher nicht frei fließend und nicht pumpfähig. Diese Produkte werden in der Regel durch Erhitzen mit konventionellen Quellen erzeugt und eignen sich nicht als Komplexbildner.

Die erfindungsgemäß eingesetzten Verbindungen der Formel (I) liegen häufig als Flüssigkeiten vor, insbesondere als transparente Flüssigkeiten mit einer Viskosität bei 25°C von mindestens 100 mPas, gemessen mit dem Rotationsviskosimeter (Brookfield-Viskosimeter).

Die erfindungsgemäß eingesetzten Verbindungen der Formel (I) liegen im Allgemeinen als Stoffgemische vor. Die bevorzugten von Zitronensäure und Ethylenglykol abgeleiteten Stoffgemische enthalten beispielsweise Ethylenglykoldizitronen-säureester der nachfolgenden Formel

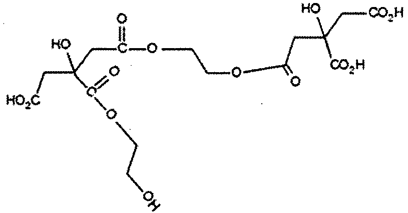

Dieser Ester eignet sich hervorragend zur Komplexbildung mit Metallionen, insbesondere mit Erdalkalimetallionen. Ein Komplex mit Calcium wird nachstehend gezeigt

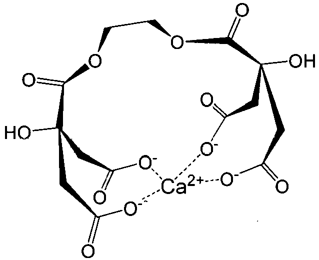

Es liegen aber auch andere Zitronensäureester im Gemisch vor, beispielsweise Verbindungen mit einer freien Carboxylgruppe anstelle der Ethylenglykolestergruppe -COO-CH₂-CH₂-OH oder Verbindungen, in denen weitere Zitronensäureeinheiten über deren Carboxylgruppe an die Hydroxylgruppe der Alkylenglykoleinheit gebunden sind.

Darüber hinaus können die Gemische auch noch geringe Anteile an nicht umgesetzten Ausgangsmaterialien, also freie Diole und/oder Hydroxycarbonsäuren oder gegebenenfalls Carbonsäuren, enthalten.

Die Gemische enthaltend unterschiedliche Polyester der Formel (I) sind in der Regel bei 25 °C flüssig. Die Viskosität dieser Gemische beträgt vorzugsweise 0,1 bis 10.000 mPa*s bei 20 °C, gemessen mit dem Brookfield-Viskosimeter (Spindeln 1 bis 7, je nach Viskositätsbereich; Schergeschwindigkeit 5 Umdrehungen/Minute), vorzugsweise 1 bis 7500 mPa*s und ganz besonders bevorzugt 100 bis 2000 mPas.

Die erfindungsgemäß eingesetzten Polyester werden durch Umsetzung von aliphatischen Hydroxycarbonsäuren mit ausgewählten aliphatischen (Poly)alkylenglykolen hergestellt. Bei letzteren handelt es sich um Alkylenglykole mit zwei, drei oder vier Kohlenstoffatomen oder um Poly-Alkylenglykole mit zwei, drei oder vier Wiederholeinheiten davon. Diese Verbindungen weisen im Allgemeinen die nachfolgende Struktur auf

HO-(CₙH₂ₙ-O)ₘ-H

worin n und m die oben definierte Bedeutung besitzen.

Bevorzugt kommen Ethylenglykole oder Propylenglykole zum Einsatz, also Verbindungen der obigen Formel, in denen n 2 oder 3 ist.

Besonders bevorzugt kommen (Poly)-ethylenglykole zum Einsatz, also Verbindungen der obigen Formel, in denen n 2 ist.

Ganz besonders bevorzugt kommen Ethylenglykol oder Diethylenglykol zum Einsatz, also Verbindungen der obigen Formel, in denen n=2 ist und m=1 oder 2 bedeutet.

Äußerst bevorzugt wird Ethylenglykol eingesetzt, also eine Verbindung der obigen Formel, in der n=2 ist und m=1 bedeutet.

Bevorzugt werden Verbindungen der Formel (I) eingesetzt, bei denen n=2 oder 3 ist, und m=1 oder 2 bedeutet. Diese Verbindungen leiten sich von Ethylenglykol, Propylenglykol, Di-ethylenglykol oder Di-propylenglykol ab.

Besonders bevorzugt werden Verbindungen der Formel (I) eingesetzt, bei denen n=2 ist, und m=1 bedeutet. Diese Verbindungen leiten sich von Ethylenglykol ab.

Zu den bei der Herstellung der erfindungsgemäß eingesetzten Verbindungen der Formel (I) verwendeten aliphatischen Hydroxycarbonsäuren zählen beliebige Typen. Es kann sich um aliphatische Hydroxycarbonsäuren mit zwei, drei oder vier oder fünf Carboxylgruppen handeln. Die aliphatischen Hydroxycarbonsäuren können ein bis vier Hydroxygruppen aufweisen. Eine Hydroxylgruppe kann sich mit einer Carboxylgruppe an einem gemeinsamen Kohlenstoffatom befinden, oder die Hydroxylgruppe befindet sich in alpha-, beta- oder anderer Position zu einer Carboxylgruppe. Die Carboxylgruppen befinden sich im Allgemeinen an unterschiedlichen Kohlenstoffatomen des aliphatischen Restes. Liegen mehrere Hydroxylgruppen vor, so befinden sich diese an unterschiedlichen Kohlenstoffatomen des aliphatischen Restes. Der aliphatische Rest weist in der Regel ein bis acht, vorzugsweise zwei bis vier Kohlenstoffatome auf.

Vorzugsweise leiten sich Verbindungen der Formel (I) ab von Äpfelsäure, Milchsäure, Tartronsäure, Weinsäure, Isozitronensäure, Zitronensäure, Acetylzitronensäure, Weinsäure oder Schleimsäure ab, insbesondere von Isozitronensäure oder Zitronensäure, und ganz besonders bevorzugt von Zitronensäure.

Bei der Herstellung der Verbindungen der Formel (I) können auch aliphatische Hydroxycarbonsäuren gegebenenfalls in Kombination mit aliphatischen Carbonsäuren eingesetzt werden. Anstelle der aliphatischen Hydroxycarbonsäuren oder der aliphatischen Carbonsäuren können auch deren esterbildende Derivate, wie beispielsweise Ester, Anhydride, Halogenide oder deren Salze, eingesetzt werden.

Bevorzugt werden bei der Herstellung der Verbindungen der Formel (I) ausschließlich ein oder mehrere aliphatische Hydroxycarbonsäuren und/oder deren reaktiven Derivate und/oder deren Salze eingesetzt.

Zu den bei der Herstellung der erfindungsgemäß eingesetzten Verbindungen der Formel (I) gegebenenfalls verwendeten aliphatischen Carbonsäuren zählen beliebige Typen. Es kann sich um aliphatische Carbonsäuren mit zwei, drei oder vier oder fünf Carboxylgruppen handeln. Die Carboxylgruppen befinden sich dabei im Allgemeinen an unterschiedlichen Kohlenstoffatomen des aliphatischen Restes. Der aliphatische Rest weist in der Regel ein bis acht, vorzugsweise zwei bis vier Kohlenstoffatome auf.

Beispiele für aliphatische Carbonsäuren sind Oxalsäure, Malonsäure, Bernsteinsäure, Glutarsäure, Adipinsäure, Pimelinsäure, Korksäure, Azelainsäure, Sebacinsäure, Carballylsäure, Butan-1,2,4-tricarbonsäure oder Octrinsäure. Bei der Herstellung der Verbindungen der Formel (I) können auch unterschiedliche aliphatische Carbonsäuren in Kombination mit aliphatischen Hydroxycarbonsäuren eingesetzt werden oder mit Derivaten von aliphatischen Hydroxycarbonsäuren, wie deren Estern oder deren Salzen.

Die Reste R¹ und R⁴ können Alkyl bedeuten. Dabei handelt es sich um Alkylgruppen mit ein bis sechs Kohlenstoffatomen, die geradkettig oder verzweigt sein können. Bevorzugt sind Methyl und Ethyl.

Die Reste R¹ und R⁴ können Cycloalkyl bedeuten. Dabei handelt es sich um Cycloalkylgruppen mit drei bis neun Ringkohlenstoffatomen, bevorzugt fünf bis sieben Ringkohlenstoffatomen. Besonderst bevorzugt ist Cyclohexyl.

Die Reste R¹ und R⁴ können Aryl bedeuten. Dabei handelt es sich um aromatische Kohlenwasserstoffreste mit fünf bis zehn Ringkohlenstoffatomen. Bevorzugt ist Phenyl.

Die Reste R¹ und R⁴ können Alkylaryl bedeuten. Dabei handelt es sich um Arylgruppen, die mit ein oder zwei Alkylgruppen substituiert sind. Bevorzugt ist Tolyl.

Die Reste R¹ und R⁴ können Aralkyl bedeuten. Dabei handelt es sich um Arylgruppen, die über eine Alkylengruppe mit der Carboxylgruppe verbunden sind. Bevorzugt ist Benzyl.

Bevorzugte Reste R¹ und R⁴ sind Wasserstoff, Metallkationen, Ammoniumkationen oder Reste der Formel -(CₙH₂ₙ-O)ₘ-H.

Besonders bevorzugte Reste R¹ und R⁴ sind Wasserstoff, Kationen von Alkalimetallen, Kationen von Erdalkalimetallen, quarternäre Ammoniumkationen oder Reste der Formel -(CₙH₂ₙ-O)ₘ-H, insbesondere Reste der Formel -C₂H₄-OH.

Die Reste R² und R³ sind aliphatische Kohlenwasserstoffreste mit ein bis acht, vorzugsweise von zwei bis vier Kohlenstoffatomen. R² und R³ können geradkettig oder verzweigt sein. Die Wertigkeit eines Restes R², also die Anzahl der kovalenten Bindungen, welche diesen Rest mit den übrigen Gruppen des Moleküls verbinden, beträgt a+c+1. In Abhängigkeit von der Größe der Indizes a und c kann die Wertigkeit von R² also zwischen 2 und 9 liegen. Die Wertigkeit eines Restes R³, beträgt b+d+1. In Abhängigkeit von der Größe der Indizes b und d kann die Wertigkeit von R³ also zwischen 3 und 9 liegen. Dabei können nicht alle der prinzipiell möglichen Reste R² oder R³ eine Wertigkeit im Bereich von 2 bis 9 oder im Bereich von 3 bis 9 aufweisen, da aufgrund der Vierbindigkeit des Kohlenstoffs die Anzahl der freien möglichen Valenzen im Einzelfall kleiner sein kann. Ein Rest R² mit z.B. nur einem Kohlenstoffatom kann daher nur Wertigkeiten im Bereich von 2 bis 4 annehmen. Dem Fachmann sind diese Zusammenhänge bekannt.

Bevorzugt werden zweiwertige Reste R² mit der Formel -CₒH₂ₒ- oder
dreiwertige Reste R² mit der Formel -CₚH₂ₚ₋₁< oder
vierwertige Reste R² mit der Formel >C_{q}H_{2q-2}<,
worin o eine ganze Zahl von 2 bis 4 ist, vorzugsweise von 2 bis 3 und besonders bevorzugt 2 bedeutet,
p eine ganze Zahl von 1 bis 4 ist, vorzugsweise von 1 bis 3 und besonders bevorzugt 1 oder 2 bedeutet, und
q eine ganze Zahl von 2 bis 4 ist, vorzugsweise von 2 bis 3 und besonders bevorzugt 3 bedeutet.

Besonders bevorzugt werden Verbindungen der Formel (I), bei denen R² und R³ Reste sind, die sich ableiten von Äpfelsäure, Milchsäure, Tartronsäure, Weinsäure, Isozitronensäure oder Zitronensäure nach dem Entfernen der Carboxylgruppen und der Hydroxygruppe.

Diese besonders bevorzugten Reste R² und R³ haben die Strukturen der Formeln (Ib), (Ic),(Id), (le), (If) oder (Ig)

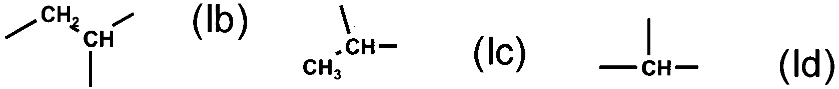

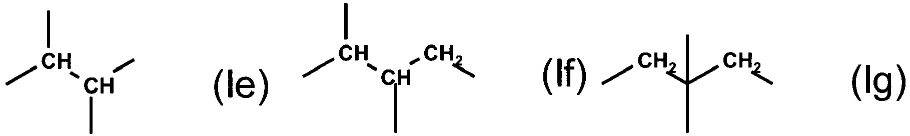

Ganz besonders bevorzugt werden Verbindungen der Formel (I) eingesetzt, bei denen a und b 2 sind, R² und R³ aliphatische Kohlenwasserstoffreste mit drei Kohlenstoffatomen sind, insbesondere von Zitronensäure abgeleitete aliphatische Kohlenwasserstoffreste, und R¹ und R⁴ Wasserstoff, Kationen von Alkalimetallen, Kationen von Erdalkalimetallen, quarternäre Ammoniumkationen oder Reste der Formel -(CₙH₂ₙ-O)ₘ-H bedeuten.

Ganz besonders bevorzugt wird die Verbindung der nachstehenden Formel (II) oder deren Alkali- oder Erdalkalisalze oder Teilneutralisate davon eingesetzt

HO-C(CH₂-COOH)₂-COO-C₂H₄-OOC-COH-(CH₂COOH)₂ (II).

Die Verbindungen der Formel (II) lassen sich auch in Form der Formel (IIa) darstellen.

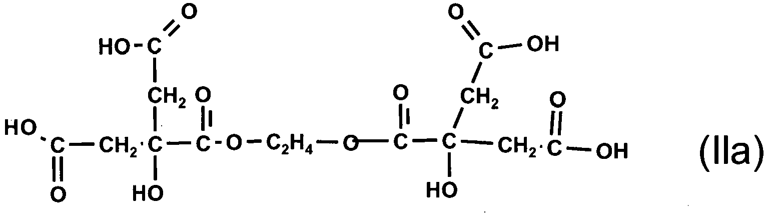

Besonders bevorzugt eingesetzt werden darüber hinaus Gemische enthaltend unterschiedliche Verbindungen der Formel (I), die bei 25°C flüssig sind.

Bevorzugt werden weiterhin Verbindungen der Formel (I) eingesetzt, die einen Rest der Formel (R¹OOC)ₐ-R²(OH)-COO- und einen Rest der Formel (R⁴OOC)_{b}-R³(OH)-COO- aufweisen, wobei diese Reste die gleiche Bedeutung besitzen.

Die Verbindungen der Formel (I) können durch die Veresterung von aliphatischen Hydroxycarbonsäuren oder deren Ester bildenden Derivaten, wie z.B. deren Alkylestern, mit ausgewählten Diolen bei erhöhter Temperatur hergestellt werden, wobei der Temperaturanstieg in der Reaktionsmischung impulsartig aber temperaturkontrolliert erfolgen muss. Unter diesen Bedingungen bilden sich lediglich kleine Moleküle und es erfolgt keine Polymerisation oder Zersetzung der eingesetzten Komponenten. Gegebenenfalls kann das Reaktionsgemisch zusätzlich aliphatische Carbonsäuren oder deren Ester bildende Derivate enthalten.

Die erfindungsgemäß eingesetzten Verbindungen der Formel (I) lassen sich durch ein Verfahren herstellen, dass folgende Maßnahmen umfasst:
i) Vorlage von Hydroxycarbonsäuren der Formel (V) oder deren esterbildenden Derivaten, wie deren Alkylestern, und gegebenenfalls von Carbonsäuren oder Hydroxycarbonsäuren der Formel (IV) oder deren esterbildenden Derivaten, wie deren Alkylestern, und von Alkylenglykol der Formel (VI)

   (R¹OOC)ₐ-R²(OH)_{c}-COOR¹ (IV)

   R⁴OOC-R³(OH)_{d}(COOR⁴)_{b} (V)

   OH-(CₙH₂ₙ-O)ₘ-H (VI)

   worin R¹, R², R³, R⁴, a, b, c, d, n und m die weiter oben definierte Bedeutung besitzen,
ii) Erhitzen der in Schritt i) erhaltenen Mischung auf mindestens 90°C für eine Zeitspanne von 0,1 Millisekunden bis 60 Minuten, und
iii) Abkühlen der in Schritt ii) erhaltenen Produktmischung auf 25 °C oder darunter innerhalb einer Zeitspanne von 1 Sekunde bis 60 Minuten.

Das Zuführen der erforderlichen Heizleistung kann durch jede beliebige Vorrichtung erfolgen, welche in der Lage ist, kurzzeitig hohe Mengen an Heizleistung in das Reaktionsgemisch einzutragen. Wichtig ist hierbei eine Begrenzung der Reaktionstemperatur auf solche Werte, dass dadurch eine Zersetzung der Reaktanten vermieden wird. Beispiele für geeignete Vorrichtungen sind Wärmetauscher, insbesondere Rekuperatoren, oder elektromagentische Strahlung im Mikrowellenband.

Als Rekuperatoren können die bekannten Typen eingesetzt werden. Beispiele dafür sind Plattenwärmeüberträger, Kapillarwärmetauscher, Mikroreaktoren, Spiralwärme-überträger, Rohrbündelwärmeübertrager, U-Rohr-Wärmeübertrager, Mantelrohrwärmeüberträger, Heizregister oder Gegenstrom-Wärmeüberträger.

Bevorzugt wird das Reaktionsgemisch in der Heizzone mit hoher Heizleistung durch Erhitzen mit elektromagentischer Strahlung im Mikrowellenband oder mit einem Wärmetauscher beaufschlagt.

Besonders bevorzugt ist die Heizzone in Form eines druckfesten, mikrowellentransparenten Rohres ausgestaltet, das sich in einem geeignet dimensionierten Hohlraumresonator befindet, welcher in der Lage ist, ein elektromag-netisches Feld, vorzugsweise im Mikrowellenband, geeigneter Feldstärke zu erzeugen, mit dessen Hilfe das Reaktionsgut durch dielektrische Heizmechanismen erhitzt wird.

Die eingesetzte elektromagnetische Strahlung weist vorzugsweise eine Frequenz im Bereich von 300 MHz bis 30 GHz auf, insbesondere eine Frequenz von 915 MHz, 2,45 GHz oder 5,8 GHz.

Durch die kurzzeitige Einwirkung von hohen Temperaturen auf das Reaktionsgemisch finden in diesem sehr schnell und gleichzeitig Ver- und Umesterungsreaktionen statt. Dabei wird der größte Teil der vorhandenen Hydroxycarbonsäuren und Diole verestert, wobei bei Hydroxycarbonsäuren mit mehreren Carboxylgruppen häufig ein Teil der Carboxylgruppen unverestert bleibt.

Bevorzugt wird ein Verfahren, bei dem das Erhitzen in Schritt ii) durch Einstrahlung von Mikrowellenstrahlung erfolgt.

Die Reaktionstemperatur in Schritt ii) liegt im Allgemeinen im Bereich von 90 bis 190 °C, vorzugsweise von 120 bis 180 °C und ganz besonders bevorzugt von 140 bis 160°C.

Ganz besonders bevorzugt wird ein Verfahren, bei dem die Mischung in Schritt ii) auf eine Temperatur zwischen 140 und 160°C erhitzt wird und bei dem die Zeitspanne des Erhitzens 1 bis 120 Sekunden beträgt.

Das oben beschriebene Verfahren kann ansatzweise oder bevorzugt kontinuierlich durchgeführt werden.

Bei der Durchführung des Verfahrens werden die Alkylenglykole vorzugsweise im molaren Überschuss zur Hydroxycarbonsäure eingesetzt.

Üblicherweise beträgt das molare Verhältnis von Hydroxycarbonsäure zu Alkylenglykol 1 : 10 bis 10 : 1, vorzugsweise 5 : 1 bis 1 : 5, und besonders bevorzugt 1 : 1 bis 1 : 3.

Das Verfahren kann in unterschiedlichen Reaktionsmischungen durchgeführt werden. Beispiele dafür sind Emulsionen oder Lösungen. Bevorzugt wird das Verfahren in Lösung durchgeführt. Im Verfahren enhält das Reaktionsgemisch gemäß Verfahrensschritt i) Verbindungen der Formeln (V) und gebenenfalls der Formel (IV) oder deren esterbildende Derivate und Verbindungen der Formel (VI).

Als Lösungsmittel kommen alle Flüssigkeiten in Frage, in denen sich die Reaktanten lösen und die unter den Reaktionsbedingungen im Wesentlichen inert sind. Beispiele dafür sind aprotische polare organische Lösungsmittel.

In einer bevorzugten Ausführungsform dient das eingesetzte Alkylenglykol gleichzeitig als Lösungsmittel für den entstehenden Oligoester.

Das Verfahren kann mit oder ohne den Einsatz von Ver- oder Umesterungskatalysatoren durchgeführt werden. Beispiele für Ver- oder Umesterungskatalysatoren sind saure Katalysatoren oder deren Gemische. Dabei kann es sich um anorganische, metallorganische und/oder um organische saure Verbindungen handeln. Als saure anorganische Katalysatoren im Sinne der vorliegenden Erfindung sind Mineralsäuren einsetzbar, beispielsweise Salzsäure, Borsäure, Salpetersäure, Schwefelsäure, Phosphorsäure, Phosphonsäure oder hypophosphorige Säure; außerdem sind saure Salze einsetzbar, wie Aluminiumsulfathydrat, Alaun, saures Kieselgel oder saures Aluminiumhydroxid. Weitere saure anorganische Katalysatoren sind beispielsweise Aluminiumverbindungen der allgemeinen Formel Al(OR)₃ und Titanate der allgemeinen Formel Ti(OR)₄, wobei die Reste R jeweils gleich oder verschieden sein können und unabhängig voneinander gewählt sind aus C₁-C₁₀-Alkylresten, beispielsweise Methyl, Ethyl, n-Propyl, isoPropyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, sec.-Hexyl, n-Heptyl, n-Octyl, 2-Ethylhexy, n-Nonyl oder n-Decyl, aus C₃-C₁₂-Cycloalkylresten, beispielsweise Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl. Bevorzugt sind die Reste R in Al(OR)₃ bzw. Ti(OR)₄ jeweils gleich und gewählt aus Isopropyl, Butyl und 2-Ethylhexyl.

Bevorzugte saure metallorganische Katalysatoren sind gewählt aus Dialkylzinnoxiden (R³)₂SnO, wobei R³ wie oben stehend definiert ist. Ein besonders bevorzugter Vertreter für saure metallorganische Katalysatoren ist Di-n-butylzinnoxid, das als sogenanntes Oxo-Zinn oder als Fascat<(R)>-Marken kommerziell erhältlich ist.

Bevorzugte saure organische Katalysatoren sind organische Verbindungen, die saure Gruppen enthalten, beispielsweise Phosphatgruppen, Phosphonsäuregruppen, Sulfonsäuregruppen, Sulfatgruppen oder Carbonsäuregruppen. Besonders bevorzugte Sulfonsäuren enthalten mindestens eine Sulfonsäuregruppe und mindestens einen gesättigten oder ungesättigten, linearen, verzweigten und/oder cyclischen Kohlenwasserstoffrest mit 1 bis 40 C-Atomen und bevorzugt mit 1 bis 24 C-Atomen. Insbesondere bevorzugt sind aromatische Sulfonsäuren und speziell alkylaromatische Monosulfonsäuren mit einem oder mehreren C₁-C₂₈-Alkylresten und insbesondere solche mit C₁-C₂₂-Alkylresten.

Bevorzugte Beispiele sind Methansulfonsäure, Butansulfonsäure, Benzolsulfonsäure, p-Toluolsulfonsäure, Xylolsulfonsäure, 2-Mesitylensulfonsäure, 4-Ethylbenzolsulfonsäure, Isopropylbenzolsulfonsäure, 4-Butylbenzolsulfonsäure, 4-Octylbenzolsulfonsäure, Dodecylbenzolsulfonsäure, Didodecylbenzolsulfonsäure, Naphthalinsulfonsäure.

Besonders bevorzugt für die Durchführung des Verfahrens sind Borsäure, Phosphorsäure, Polyphosphorsäure und Polystyrolsulfonsäuren.

Insbesondere bevorzugt sind Titanate der allgemeinen Formel Ti(OR)₄ und speziell Titantetrabutylat und Titantetraisopropylat.

In einer weiteren Ausführungsform werden im Verfahren saure, feste Katalysatoren eingesetzt. Beispiele dafür sind Zeolithe, Kieselgel, saure Schichtsilikate, wie Montmorillonit, und organische Ionenaustauscher.

Vorzugsweise wird das Verfahren ohne den Einsatz von Ver- oder Umesterungskatalysatoren durchgeführt.

Die Katalysatoren werden typischerweise in Mengen von bis zu 10 Gew. %, bezogen auf die Gesamtmasse des Reaktionsgemisches verwendet, vorzugsweise in Mengen von 0,01 bis 10 Gew.-% und besonders bevorzugt von 0,02 bis 2 Gew.-%.

Das Reaktionsgemisch wird durch einen Reaktor geleitet. Dabei erfährt das Reaktionsgemisch in einer Heizzone durch Zufuhr von Heizleistung eine starke Erwärmung. Dieses kann durch physischen Kontakt mit einer wärmeren Wand durch Wärmeübertragung erfolgen oder durch Wechselwirkung polarer oder ionischer Moleküle mit elektromagnetischen Feldern, beispielsweise mit Wellenlängen im Zentimeterbereich (Mikrowelle)

Das Reaktionsgemisch wird in der Heizzone für eine Zeitspanne von bis zu 60 Minuten, typischerweise von 0,1 Millisekunden bis zu 60 Minuten, bevorzugt von 1 Sekunde bis 10 Minuten, und ganz besonders bevorzugt von 1 Sekunde bis zu 2 Minuten mit einer ausreichend hohen Heizleistung beaufschlagt. Dabei erfährt das Reaktionsgemisch eine starke Temperaturerhöhung und weist beim Verlassen der Heizzone eine Temperatur zwischen 90°C und 190°C, bevorzugt zwischen 120°C und 180°C und besonders bevorzugt zwischen 140°C und 160°C auf, gemessen mittels Temperatursensor PT100 unmittelbar nach Verlassen der Heizzone.

Das Reaktionsgemisch kann im Reaktor unter Unterdruck, Atmosphärendruck oder insbesondere unter Überdruck vorliegen. Vorzugsweise beträgt der Druck im Reaktor 0 bis 1000 bar absolut, besonders bevorzugt 1 mbar bis 200 bar, absolut, ganz besonders bevorzugt 50 mbar bis 20 bar absolut und ganz besonders bevorzugt zwischen 1 und 20 bar absolut. Der Druck im Reaktor ist insbesondere so zu wählen, dass sowohl das Reaktionsgemisch als auch die während der Reaktion entstehenden Kondensate im Reaktor in flüssigem Zustand vorliegen. In dieser Variante wird das Reaktionsgemisch in der Reaktionszone effizient aufgeheizt, insbesondere für den Fall, dass dazu elektromagnetische Strahlung, wie beispielsweise Mikrowellenstrahlung, eingesetzt wird.

Die Verweilzeit in der Heizzone wird durch die Auswahl einer geeigneten Stömungsgeschwindigkeit des Reaktionsgemisches durch diese Zone eingestellt. Eine weitere bevorzugte Möglichkeit im Sinne der Erfindung zur Anpassung der Verweilzeit ist eine geeignete Auswahl der Apparategröße.

In einer Ausführungsform des Verfahrens schließt sich der Heizzone des Reaktors eine Verweilstrecke an. Das aus dem Reaktionsgemisch entstandene Produktgemisch kann nach der Heizzone in dieser Verweilstrecke für eine Verweildauer von bis zu 60 Minuten, bevorzugt von 1 bis 600 Sekunden, besonders bevorzugt von 1 bis 120 Sekunden, verbleiben.

Das Zuführen der erforderlichen Heizleistung in der Heizzone kann durch jede beliebige Vorrichtung erfolgen, welche in der Lage ist, kurzzeitig hohe Mengen an Heizleistung in das Reaktionsgemisch einzutragen. Beispiele für geeignete Vorrichtungen wurden bereits weiter oben beschrieben.

Durch die kurzzeitige Einwirkung von hohen Temperaturen und Drucken auf das Reaktionsgemisch finden in diesem sehr schnell Veresterungs- und gegebenenfalls Umesterungsreaktionen statt. Dabei wird ein Teil der vorhandenen Alkohole und Carbonsäuren in Carbonsäureester mit vergleichsweise geringem Molekular-gewicht umgewandelt und es werden je nach Ausgangssubstanz Alkohole und/oder Reaktionswasser freigesetzt.

Das Abkühlen des heißen Produktgemisches in Schritt iii) kann in der Verweilstrecke und/oder in einer der Verweilzone oder der Reaktionszone nachgeschalteten Abkühlstrecke erfolgen. Vorzugsweise wird das heiße Produktgemisch rasch abgekühlt, um weitere Umsetzungen zu vermeiden.

Das erhaltene Produktgemisch kann als solches ohne weitere Aufarbeitung mit anderen Substanzen kombiniert werden oder es kann vor einer Weiterverarbeitung aufgearbeitet werden.

Nach Schritt iii) kann als Aufarbeitung beispielsweise eine Trocknung und/oder Neutralisation des Produktgemisches und/oder ein Abtrennen von festen Bestandteilen erfolgen.

Das gegebenenfalls aufgearbeitete Produktgemisch kann durch Aufbringen auf einen festen Träger und/oder durch Granulieren zusammen mit anderen Substanzen weiterverarbeitet werden.

Versuche haben gezeigt, dass die erfindungsgemäß eingesetzten Verbindungen der Formel (I) sich als außerordentlich gute Komplexbildner für Metallkationen, insbesondere für Erdalkalimetallkationen, wie Mg²⁺ und Ca²⁺, eignen

Die Erfindung betrifft daher auch die Verwendung der Verbindungen der Formel (I) als Komplexbildner in Wasch- und Reinigungsmitteln, bei der Erdölförderung oder zur Wasserenthärtung.

Als Wasch- und Reinigungsmittel kommen insbesondere Mittel für die Reinigung von Geschirr in Frage, ganz besonders solche, die für den Einsatz in automatischen Geschirrspülern geeignet sind.

Für den Einsatz in Wasch- und Reinigungsmitteln kommen die Verbindungen der Formel (I) üblicherweise in der Form von Granulaten in Kombination mit anderen Bestandteilen von Wasch- und Reinigungsmitteln zum Einsatz.

Die Leistung von Bleichmitteln in Wasch- und Reinigungsmitteln kann deutlich vergrößert werden, wenn ein als Persauerstoffverbindung eingesetztes Bleichmittel mit einer Kombination von Bleichkatalysator mit Bleichaktivator in Kontakt gebracht wird. Hierbei wird die bleichende Wirkung des Katalysators durch die aus dem Aktivator gebildete Peroxycarbonsäure wirksam unterstützt. Zugleich trägt die Peroxycarbonsäure signifikant zur Keimabtötung auf dem zu reinigenden Gut bei, verbessert den Geruch der Waschlauge und unterbindet die Ausbildung eines Biofilms in der Wasch- oder Spülmaschine. Die Kombination von Bleichkatalysatoren und/oder Bleichaktivatoren ist deshalb sinnvoll zur Steigerung der Bleichwirkung und zur Gewährleistung der Hygiene bei der Verwendung von Bleichmitteln in Wasch- und Reinigungsmitteln.

Bevorzugte erfindungsgemäße Wasch- und Reinigungsmittel, insbesondere die Mittel für die Reinigung von Geschirr, enthalten die erfindungsgemäß eingesetzten Verbindungen der Formel (I) in Mengen zwischen 0,1 und 10 Gew.-%, vorzugsweise in Mengen zwischen 0,2 und 8 Gew.-% und besonders bevorzugt in Mengen zwischen 0,5 und 6 Gew.-%. Dabei beziehen sich die Prozentangaben auf das Gesamtgewicht des Wasch- und Reinigungsmittels.

Die erfindungsgemäßen Wasch- und Reinigungsmittel, die als Granulate, pulver- oder tablettenförmige Feststoffe aber auch in flüssiger oder pastöser Form vorliegen können, können außer den erfindungsgemäß eingesetzten Verbindungen der Formel (I) im Prinzip alle bekannten und in derartigen Mitteln üblichen Inhaltsstoffe enthalten.

Die erfindungsgemäßen Wasch- und Reinigungsmittel können insbesondere Buildersubstanzen, Persauerstoffverbindungen, Enzyme, Alkaliträger, oberflächenaktive Tenside, pH-Regulatoren, organische Lösungsmittel und weitere Hilfsstoffe, wie Glaskorrosionsinhibitoren, Silberkorrosionsinhibitoren und Schaumregulatoren enthalten. Die erfindungsgemäßen Granulate sind sowohl zum Einsatz in phosphathaltigen als auch insbesondere in phosphatfreien Formulierungen geeignet.

Besonders bevorzugte Wasch- und Reinigungsmittel, insbesondere Mittel für die Reinigung von Geschirr, enthalten
i) 5 bis 65 Gew.-%, vorzugsweise 10 bis 60 Gew.-% einer wasserlöslichen Builderkomponente,
ii) 5 bis 20 Gew.-%, vorzugsweise 8 bis Gew.-%, einer Persauerstoffverbindung,
iii) 0,5 bis 25 Gew.-% einer Verbindung der Formel (I), und
iv) 0 bis 50 Gew.-% weitere Zusatzstoffe wie Enzyme, Alkaliträger, oberflächenaktive Tenside, pH-Regulatoren, organische Lösungsmittel oder weitere Hilfsstoffe, wie Glaskorrosionsinhibitoren, Silberkorrosionsinhibitoren und Schaumregulatoren, jeweils bezogen auf das Gesamtgewicht des Wasch- und Reinigungsmittels.

Ein derartiges Mittel ist insbesondere niederalkalisch, d. h. seine 1-gewichtsprozentige wässrige Lösung weist einen pH-Wert im Bereich von 8 bis 11,5 und vorzugsweise von 8 bis 11 auf.

Mögliche Inhaltsstoffe von Wasch- und Reinigungsmitteln sind in der Patentliteratur hinlänglich beschrieben, beispielsweise in der WO 2018/210442 A1.

Beispiele für bevorzugte wasserlösliche Builderkomponenten in den erfindungsgemäßen Wasch- und Reinigungsmitteln sind organische Polymere nativen oder synthetischen Ursprungs vom Typ der Polycarboxylate, die insbesondere in Hartwasserregionen als Co-Builder wirken. In Betracht kommen beispielsweise Polyacrylsäuren und Copolymere aus Maleinsäureanhydrid und Acrylsäure sowie die Natriumsalze dieser Polymersäuren. Handelsübliche Produkte sind zum Beispiel Sokalan^{®} CP 5, CP 10 und PA 30 der Firma BASF. Zu den als Co-Builder brauchbaren Polymeren nativen Ursprungs gehören beispielsweise oxidierte Stärke und Polyaminosäuren wie Polyglutaminsäure oder Polyasparaginsäure. Weitere mögliche wasserlösliche Builder-komponenten sind natürlich vorkommende Hydroxycarbonsäuren, wie zum Beispiel Mono-, Dihydroxybernsteinsäure, alpha-Hydroxypropionsäure und Gluconsäure. Zu den bevorzugten organischen wasserlöslichen Builderkomponenten gehören die Salze der Citronensäure, insbesondere Natriumcitrat. In Abhängigkeit vom letztlich in den erfindungsgemäßen Wasch- und Reinigungsmitteln eingestellten pH-Wert können auch die zu den genannten Co-Builder-Salzen korrespondierenden Säuren vorliegen. Insbesonders bevorzugte Builderkomponenten in phosphatfreien Formulierungen sind Methylglycindiacetat (MDGA, z. B. Trilon^{®} M, BASF), L-Glutaminsäure, N,N, (biscarboxymethyl)- tertra Natriumsalz(GLDA, Dissolvine^{®} DL, Akzo Nobel), Natrium-Polyaspartate (Baypure^{®}, Lanxess) oder Salze der Iminodibernsteinsäure (Baypure^{®}, Lanxess).

Beispiele für bevorzugte Persauerstoffverbindungen in den erfindungsgemäßen Wasch- und Reinigungsmitteln sind Perborate und Percarbonate, insbesondere die entsprechenden Natriumsalze dieser Verbindungen.

Zu den in erfindungsgemäßen Wasch- und Reinigungsmitteln gegebenenfalls enthaltenen Enzymen gehören Proteasen, Amylasen, Pullulanasen, Cutinasen und/oder Lipasen. Die verwendeten Enzyme können an Trägerstoffen adsorbiert und/oder in Hüllsubstanzen eingebettet sein, um sie gegen vorzeitige Inaktivierung zu schützen. Sie sind in den erfindungsgemäßen Wasch- und Reinigungsmitteln typischerweise in Mengen bis zu 10 Gew.-% und bevorzugt in Mengen von 0,05 bis 5 Gew.-% enthalten, wobei insbesondere bevorzugt gegen oxidativen Abbau stabilisierte Enzyme eingesetzt werden.

Vorzugsweise enthalten die erfindungsgemäßen Wasch- und Reinigungsmittel, insbesondere die Mittel für die Reinigung von Geschirr, übliche Alkaliträger wie zum Beispiel Alkalisilikate, Alkalicarbonate und/oder Alkalihydrogencarbonate. Beispiele dafür sind in der WO 2018/210442 A1 genannt. Alkaliträger können in einer Menge von bis zu 50 Gew. % und vorzugsweise von 5 bis 40 Gew. % im Wasch- und Reinigungsmittel enthalten sein.

Beispiele für bevorzugte in den erfindungsgemäßen Wasch- und Reinigungsmitteln gegebenenfalls enthaltenen Tenside sind Aniontenside, zwitterionische Tenside und vorzugsweise schwach schäumende nichtionische Tenside. Ihre Menge kann bis zu 20 Gew.-%, vorzugsweise bis zu 10 Gew.-% betragen und liegt besonders bevorzugt im Bereich von 0,5 bis 5 Gew.-%, bezogen jeweils auf das Gesamtgewicht des Wasch- und Reinigungsmittels. Beispiele für Tenside sind in der WO 2018/210442 A1 genannt.

Zur Einstellung eines gewünschten, sich durch die Mischung der übrigen Komponenten nicht von selbst ergebenden pH-Werts können die erfindungsgemäßen Wasch- und Reinigungsmittel system- und umweltverträgliche Säuren, insbesondere Zitronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure oder Alkalihydrogensulfate, oder Basen, insbesondere Ammonium- oder Alkalihydroxide, enthalten. Derartige pH-Regulatoren sind in den erfindungsgemäßen Wasch- und Reinigungsmitteln, insbesondere den Mitteln für die Reinigung von Geschirr, vorzugsweise nicht über 10 Gew.-% und besonders bevorzugt von 0,5 bis 6 Gew.-%, enthalten, bezogen jeweils auf das Gesamtgewicht des Mittels.

Beispiele für bevorzugte in den erfindungsgemäßen Wasch- und Reinigungsmitteln gegebenenfalls enthaltenen organischen Lösungsmitteln sind Alkohole mit 1 bis 4 C-Atomen, insbesondere Methanol, Ethanol, Isopropanol und tert.-Butanol, Diole mit 2 bis 4 C-Atomen, insbesondere Ethylenglykol und Propylenglykol, sowie deren Gemische und die aus den genannten Verbindungsklassen ableitbaren Ether. Derartige wassermischbare Lösungsmittel sind in den erfindungsgemäßen Wasch- und Reinigungsmitteln typischerweise in einer Menge von nicht über 20 Gew.-% und besonders bevorzugt von 1 bis 15 Gew.-% vorhanden.

Zur Unterdrückung der Glaskorrosion während des Spülganges können in den erfindungsgemäßen Wasch- und Reinigungsmitteln, insbesondere den Mitteln für die Reinigung von Geschirr, entsprechende Inhibitoren eingesetzt werden. Besonders vorteilhaft sind hier kristalline schichtförmige Silikate und/oder Zinksalze. Beispiele für Glaskorrosionsinhibitoren sind in der WO 2018/210442 A1 genannt.

In einer weiteren bevorzugten Ausführungsform weisen die erfindungsgemäßen Wasch- und Reinigungsmittel, insbesondere die Mittel für die Reinigung von Geschirr, eine Menge des kristallinen schichtförmigen Silikats von 0,1 bis 20 Gew.- %, besonders bevorzugt 0,2 bis 15 Gew.-% und insbesondere bevorzugt 0,4 bis 10 Gew.-%, jeweils bezogen auf das Gesamtgewicht des Mittels, auf.

Um einen Silberkorrosionsschutz zu bewirken, können in den erfindungsgemäßen Wasch- und Reinigungsmitteln, insbesondere in den Mitteln für die Reinigung von Geschirr, Silberkorrosionsinhibitoren eingesetzt werden. Beispiele für Silberkorrosionsinhibitoren sind in der WO 2018/210442 A1 genannt.

Die erfindungsgemäßen Wasch- und Reinigungsmittel, insbesondere die Mittel für die Reinigung von Geschirr, können als weitere Inhaltsstoffe beispielsweise aus dem Stand der Technik für derartige Mittel bekannte Sequestrierungsmittel, Elektrolyte, zusätzliche Persauerstoff-Aktivatoren, Farbstoffe oder Duftstoffe, wie z. B. Parfümöle, enthalten.

Die Herstellung der erfindungsgemäßen festen Wasch- und Reinigungsmittel, insbesondere der Mittel für die Reinigung von Geschirr, bietet keine Schwierigkeiten und kann im Prinzip in bekannter Weise, zum Beispiel durch Sprühtrocknung oder Granulation, erfolgen, wobei Persauerstoffverbindung und erfindungsgemäßes Granulat gegebenenfalls später getrennt zugesetzt werden.

Erfindungsgemäße Wasch- und Reinigungsmittel in Form wässriger oder sonstige übliche Lösungsmittel enthaltender Lösungen, insbesondere entsprechende Mittel für die Reinigung von Geschirr, werden besonders vorteilhaft durch einfaches Mischen der Inhaltsstoffe, die in Substanz oder als Lösung in einen automatischen Mischer gegeben werden können, hergestellt.

Die erfindungsgemäßen Wasch- und Reinigungsmittel, insbesondere Mittel für die Reinigung von Geschirr, liegen vorzugsweise als pulverförmige, granulare oder tablettenförmige Präparate vor, die in an sich bekannter Weise, beispielsweise durch Mischen, Granulieren, Walzenkompaktieren und/oder durch Sprühtrocknung von thermisch belastbaren Komponenten und Zumischen der empfindlicheren Komponenten, zu denen insbesondere Enzyme, Bleichmittel und der Bleichkatalysator zu rechnen sind, hergestellt werden können.

Erfindungsgemäße Mittel für die maschinelle Reinigung von Geschirr können sowohl in Haushaltsgeschirrspülmaschinen wie in gewerblichen Spülmaschinen eingesetzt werden. Die Zugabe erfolgt von Hand oder mittels geeigneter Dosiervorrichtungen. Die Anwendungskonzentrationen in der Reinigungsflotte betragen in der Regel etwa 1 bis 8 g/l, vorzugsweise 2 bis 5 g/l.

Ein maschinelles Spülprogramm wird zweckmäßig durch einige auf den Reinigungsgang folgende Zwischenspülgänge mit klarem Wasser und einem Klarspülgang mit einem gebräuchlichen Klarspülmittel ergänzt und beendet. Nach dem Trocknen erhält man beim Einsatz der erfindungsgemäßen Geschirrspülmittel ein völlig sauberes und in hygienischer Hinsicht einwandfreies Geschirr.

### Beispiele

In den folgenden Beispielen bedeuten %-Angaben Gewichtsprozent, sofern nicht explizit anders angegeben.

### Herstellungsbeispiele

### Beispiel 1: Herstellung von Oligo-Zitronensäure-Monoethylenglykol Ester

In einem 5 I Büchi-Rührautoklaven mit Rührer, Innenthermometer und Druckausgleich wurden 2,5 kg Zitronensäure (als Monohydrat) vorgelegt und mit 6,5 kg Monoethylenglykol sowie 0,05 kg Schwefelsäure versetzt.

Das so erhaltene Gemisch wurde auf 80°C angeheizt, dabei gingen alle Reaktanden vollständig in Lösung. Bei einem Arbeitsdruck von 15 bar wurde nun die Reaktionslösung kontinuierlich mit 5 I/h durch das Reaktionsrohr gepumpt und einer Mikrowellenleistung von 1,5 kW ausgesetzt, von denen 91 % vom Reaktionsgut absorbiert wurden. Die Verweilzeit des Reaktionsgemischs in der Bestrahlungszone betrug ca. 25 Sekunden. Am Ende des Reaktionsrohres hatte das Reaktionsgemisch eine Temperatur von 155 °C. Das Reaktionsgemisch wurde direkt nach Verlassen des Reaktors mit einem Intensivwärmetauscher auf Raumtemperatur abgekühlt.

### Beispiel 2 (Vergleich): Herstellung von Oligo-Zitronensäure-Glyceryl Ester

In einem 5 I Büchi-Rührautoklaven mit Rührer, Innenthermometer und Druckausgleich wurden 2,5 kg Zitronensäure (als Monohydrat) vorgelegt und mit 4,8 kg Glycerin sowie 0,05 kg Methansulfonsäure versetzt.

Das so erhaltene Gemisch wurde auf 75°C angeheizt, dabei gingen alle Reaktanden vollständig in Lösung. Bei einem Arbeitsdruck von 15 bar wurde nun die Reaktionslösung kontinuierlich mit 5 I/h durch das Reaktionsrohr gepumpt und einer Mikrowellenleistung von 1,3 kW ausgesetzt, von denen 94 % vom Reaktionsgut absorbiert wurden. Die Verweilzeit des Reaktionsgemischs in der Bestrahlungszone betrug ca. 25 Sekunden. Am Ende des Reaktionsrohres hatte das Reaktionsgemisch eine Temperatur von 160 °C. Das Reaktionsgemisch wurde direkt nach Verlassen des Reaktors mit einem Intensivwärmetauscher auf Raumtemperatur abgekühlt.

### Beispiel 3: Herstellung von Oligo-Zitronensäure-Monoethylenglykol Ester ohne Verwendung eines reaktionsbeschleunigenden Katalysators

In einem 5 I Büchi-Rührautoklaven mit Rührer, Innenthermometer und Druckausgleich wurden 2,5 kg Zitronensäure (als Monohydrat) vorgelegt und mit 6,5 kg Monoethylenglykol versetzt. Auf die Zugabe eines Katalysators wurde an dieser Stelle verzichtet.

Das so erhaltene Gemisch wurde auf 80°C angeheizt, dabei gingen alle Reaktanden vollständig in Lösung. Bei einem Arbeitsdruck von 15 bar wurde nun die Reaktionslösung kontinuierlich mit 5 I/h durch das Reaktionsrohr gepumpt und einer Mikrowellenleistung von 1,5 kW ausgesetzt, von denen 90 % vom Reaktionsgut absorbiert wurden. Die Verweilzeit des Reaktionsgemischs in der Bestrahlungszone betrug ca. 25 Sekunden. Am Ende des Reaktionsrohres hatte das Reaktionsgemisch eine Temperatur von 155 °C. Das Reaktionsgemisch wurde direkt nach Verlassen des Reaktors mit einem Intensivwärmetauscher auf Raumtemperatur abgekühlt.

### Anwendungsbeispiele

Die gemäß Herstellungsbeispielen 1, 2 und 3 hergestellten transparenten Lösungen wurden in einer Geschirrspülmittelformulierung auf Citratbasis getestet. Es wurde untersucht, ob durch den Einsatz dieser Lösungen die Ablagerung von Kalziumkarbonat auf Gläsern und anderen Geschirren verhindert werden kann.

In der nachfolgenden Tabelle wird die Zusammensetzung von Geschirrspülmitteln beschrieben, welche in den Tests eingesetzt wurden.

**Tabelle**

| **Rohstoff** | **1** | **2** | **3** | **4** |
|---|---|---|---|---|
| Tri-natrium-Citrat | 36% | 35% | 35% | 35% |
| Natriumcarbonat | 30% | 30% | 30% | 30% |
| Percarbonate | 15% | 15% | 15% | 15% |
| TAED | 5% | 5% | 5% | 5% |
| PEG1500 | 3% | 3% | 3% | 3% |
| PEG6000 | 2% | 2% | 2% | 2% |
| Sokalan PA25 | 5% | 5% | 5% | 5% |
| Lutensol TO 7 | 1% | 1% | 1% | 1% |
| Protease Blaze 100T | 2% | 2% | 2% | 2% |
| Amylase Stainzyme Evity 12T | 1% | 1% | 1% | 1% |
| Perfum, Farbstoff, etc. | 0% | 0% | 0% | 0% |
| Gemisch gemäß Herstellungsvorschrift 1 | 0% | 1% | 0% | 0% |
| Gemisch gemäß Herstellungsvorschrift 2 | 0% | 0% | 1% | 0% |
| Gemisch gemäß Herstellungsvorschrift 3 | 0% | 0% | 0% | 1% |
| **Summe** | 100% | 100% | 100% | 100% |

Die Pulverformulierungen wurden gemischt und als 20g Portionen aus der Dosierkammer dosiert. Das flüssige Gemisch aus Herstellungsbeispiel 1 bzw. 2 bzw. 3 wurde über Pipetten auf dem Pulver dosiert. Der pH-Wert, gemessen als 1 Gew.%-ige Lösung in Wasser, betrug 10,2.

Die Spülleistung wurde in einem Miele GSL 2 bei 55°C unter Verwendung von Wasser mit 21 Grad Härtegrad gemessen. Es wurden jeweils 3 Waschprogramme durchlaufen. Longdrinkgläser von Schott Zwiesel wurden hinsichtlich Film- und Fleckenbildung in einer Blackbox visuell bewertet. Es wurden Bewertungen von 1 bis 10 zugewiesen (1 = schlechtester Wert; 10 = bester Wert). Der Durchschnittswert aller Bewertungen wurde berechnet. Die Ergebnisse finden sich in der nachstehenden Tabelle.

**Tabelle**

| Formulierung | 1 | 2 | 3 | 4 |
|---|---|---|---|---|
| Film- und Fleckenbildung | 5,9 | 6,9 | 5,1 | 7,6 |

Der Glanz auf den Gläsern hatte sich deutlich verbessert, wenn ein Gemisch enthaltend Monoethylenglykol-di-Zitronensäureester hergestellt nach Herstellungsbeispiel 1 und 3 in der Geschirrspülmittel-Formulierung verwendet wurde.

Der Glanz auf den Gläsern hatte sich nicht verbessert, wenn ein Gemisch enthaltend Monoglycerin-di-Zitronensäureester hergestellt nach Herstellungsbeispiel 2 in der Geschirrspülmittel-Formulierung verwendet wurde.

## Patentansprüche

1. Wasch- und Reinigungsmittel enthaltend Verbindungen der Formel (I)
(R¹OOC)ₐ-R²(OH)_{c}-COO-(CₙH₂ₙ-O)ₘ-OC-R³(OH)_{d}(COOR⁴)_{b}, (I)
worin R¹ und R⁴ unabhängig voneinander Wasserstoff, ein Kation eines Metalls, ein Ammoniumkation, C₁-C₆-Alkyl, Cycloalkyl mit drei bis neun Ringkohlenstoffatomen, Aryl mit fünf bis zehn Ringkohlenstoffatomen, Aryl, das mit ein oder zwei Alkylgruppen substituiert ist, Aryl, das über eine Alkylengruppe mit der Carboxylgruppe verbunden ist, -(CₙH₂ₙ-O)ₘ-H oder -O-R²(COOR¹)ₐ₊₁ bedeuten,
R² und R³ unabhängig voneinander aliphatische Kohlenwasserstoffreste mit ein bis acht Kohlenstoffatomen sind,
a und b unabhängig voneinander ganze Zahlen von 1 bis 4 sind,
c eine ganze Zahl von 0 bis 4 ist,
d eine ganze Zahl von 1 bis 4 ist,
n 2, 3 oder 4 bedeutet, und
m 1, 2, 3 oder 4 ist, mit der Maßgabe, dass R¹ und R⁴ innerhalb eines Moleküls im Rahmen der gegebenen Definitionen unterschiedlich sein können.

2. Wasch- und Reinigungsmittel nach Anspruch 1, **dadurch gekennzeichnet, dass** n 2 oder 3 ist, und m 1 oder 2 bedeutet.

3. Wasch- und Reinigungsmittel nach Anspruch 2, **dadurch gekennzeichnet, dass** n 2 ist, und m 1 bedeutet.

4. Wasch- und Reinigungsmittel nach mindestens einem der Ansprüche 1 bis 3, **dadurch gekennzeichnet, dass** c und d 1 bedeuten.

5. Wasch- und Reinigungsmittel nach mindestens einem der Ansprüche 1 bis 4, **dadurch gekennzeichnet, dass** R² einen zweiwertigen Rest der Formel -CₒH₂ₒ-
oder einen dreiwertigen Rest der Formel -CₚH₂ₚ₋₁< oder einen vierwertigen Rest der Formel >C_{q}H_{2q-2}< bedeutet,
worin o eine ganze Zahl von 2 bis 4 ist, vorzugsweise von 2 bis 3 und besonders bevorzugt 2,
p eine ganze Zahl von 1 bis 4 ist, vorzugsweise von 1 bis 3 und besonders bevorzugt 1 oder 2, und
q eine ganze Zahl von 2 bis 4 ist, vorzugsweise von 2 bis 3 und besonders bevorzugt 3.

6. Wasch- und Reinigungsmittel nach mindestens einem der Ansprüche 1 bis 5, **dadurch gekennzeichnet, dass** R² und R³ Reste sind, die sich ableiten von Äpfelsäure, Milchsäure, Tartronsäure, Weinsäure, Isocitronensäure, Citronensäure, Acetylzitronensäure, Weinsäure oder Schleimsäure nach dem Entfernen der Carboxylgruppen und der Hydroxylgruppen.

7. Wasch- und Reinigungsmittel nach Anspruch 6, **dadurch gekennzeichnet, dass** R² und R³ Reste der Formel (Ib), (Ic), (Id), (le), (If) oder (Ig) sind

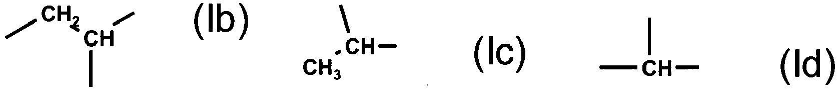

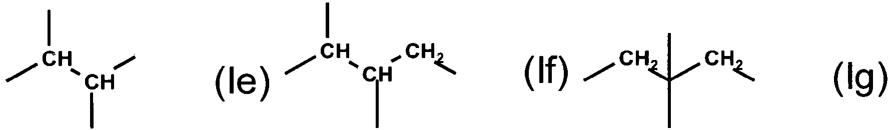

8. Wasch- und Reinigungsmittel nach Anspruch 6, **dadurch gekennzeichnet, dass** R² und R³ Reste sind, die sich ableiten von Citronensäure nach dem Entfernen der Carboxylgruppen und der Hydroxylgruppe.

9. Wasch- und Reinigungsmittel nach mindestens einem der Ansprüche 1 bis 8, **dadurch gekennzeichnet, dass** a und b 2 sind, dass R² und R³ aliphatische Kohlenwasserstoffreste mit drei Kohlenstoffatomen sind und dass R¹ und R⁴ Wasserstoff, Kationen von Alkalimetallen, Kationen von Erdalkalimetallen, quarternäre Ammoniumkationen oder Reste der Formel -(CₙH₂ₙ-O)ₘ-H sind.

10. Wasch- und Reinigungsmittel nach mindestens einem der Ansprüche 1 bis 9, **dadurch gekennzeichnet, dass** die Verbindungen der Formel (I) die Struktur der Formel (II) aufweisen oder deren Alkali- oder Erdalkalisalze oder Teilneutralisate davon
HO-C(CH₂-COOH)₂-COO-C₂H₄-OOC-COH-(CH₂COOH)₂ (II).

11. Wasch- und Reinigungsmittel nach mindestens einem der Ansprüche 1 bis 10, **dadurch gekennzeichnet, dass** die Verbindungen der Formel (I) als Gemische mehrerer Verbindungen der Formel (I) vorliegen, die bei 25°C flüssig sind.

12. Wasch- und Reinigungsmittel nach mindestens einem der Ansprüche 1 bis 11, **dadurch gekennzeichnet, dass** die Verbindungen derr Formel (I) einen Rest der Formel (R¹OOC)ₐ-R²(OH)-COO- und einen Rest der Formel (R⁴OOC)_{b}-R³(OH)-COO- aufweisen, wobei diese Reste die gleiche Bedeutung besitzen.

13. Wasch- und Reinigungsmittel nach mindestens einem der Ansprüche 1 bis 12, **dadurch gekennzeichnet, dass** dieses ein Mittel für die Reinigung von Geschirr ist.

14. Verwendung von Verbindungen der Formel (I)
(R¹OOC)ₐ-R²(OH)_{c}-COO-(CₙH₂ₙ-O)ₘ-OC-R³(OH)_{d}(COOR⁴)_{b} (I)
worin R¹, R², R³, R⁴, a, b, c, d, n und m die in Anspruch 1 definierte Bedeutuung besitzen, als Komplexbildner in Wasch- und Reinigungsmitteln, bei der Erdölförderung oder zur Wasserenthärtung.

## Claims

1. Detergent and cleaning agent containing compounds of the formula (I)
(R¹OOC)ₐ-R²(OH)_{c}-COO-(CₙH₂ₙ-O)ₘ-OC-R³(OH)_{d}(COOR⁴)_{b} (I)
wherein R¹ and R⁴ independently of one another are hydrogen, a cation of a metal, an ammonium cation, C₁-C₆-alkyl, cycloalkyl having three to nine ring carbon atoms, aryl having five to ten ring carbon atoms, aryl substituted by one or two alkyl groups, aryl linked to the carboxyl group via an alkylene group, -(CₙH₂ₙ-O)ₘ-H or -O-R²(COOR¹)ₐ₊₁,
R² and R³ independently of one another are aliphatic hydrocarbon radicals having one to eight carbon atoms,
a and b independently of one another are integers from 1 to 4,
c is an integer from 0 to 4,
d is an integer from 1 to 4,
n is 2, 3 or 4, and
m is 1, 2, 3 or 4, with the proviso that R¹ and R⁴ may be different within a molecule within the scope of the given definitions.

2. Detergent and cleaming agent according to claim 1, **characterized in that** n is 2 or 3, and m is 1 or 2.

3. Detergent and cleaning agent according to claim 2, **characterized in that** n is 2 and m is 1.

4. Detergent and cleaning agent according to at least one of claims 1 to 3, **characterized in that** c and d are 1.

5. Detergent and cleaning agent according to at least one of claims 1 to 4, **characterized in that** R² is a divalent radical of the formula -CₒH₂ₒ- or a trivalent radical of the formula -CₚH₂ₚ₋₁< or a tetravalent radical of the formula >C_{q}H_{2q-2}< in which o is an integer from 2 to 4, preferably from 2 to 3 and particularly preferred 2,
p is an integer from 1 to 4, preferably from 1 to 3 and particularly preferred 1 or 2, and
q is an integer from 2 to 4, preferably from 2 to 3 and particularly preferred 3.

6. Detergent and cleaning agent according to at least one of claims 1 to 5, **characterized in that** R² and R³ are radicals which are derived from malic acid, lactic acid, tartronic acid, tartaric acid, isocitric acid, citric acid, acetyl citric acid, tartaric acid or mucic acid after removal of the carboxyl groups and the hydroxyl groups.

7. Detergent and cleaning agent according to claim 6, **characterized in that** R² and R³ are radicals of the formula (Ib), (Ic), (Id), (le), (If) or (Ig)

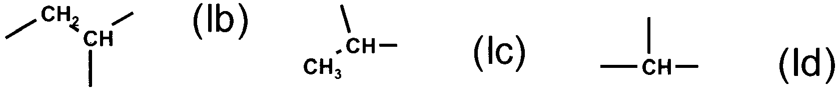

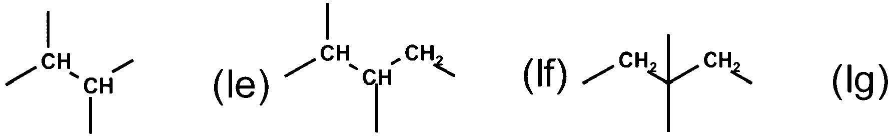

8. Detergent and cleaning agent according to claim 6, **characterized in that** R² and R³ are radicals which are derived from citric acid after removal of the carboxyl groups and the hydroxyl group.

9. Detergent and cleaning agent according to at least one of claims 1 to 8, **characterized in that** a and b are 2, **in that** R² and R³ are aliphatic hydrocarbon radicals having three carbon atoms and **in that** R¹ and R⁴ are hydrogen, cations of alkali metals, cations of alkaline earth metals, quaternary ammonium cations or radicals of the formula -(CₙH₂ₙ-O)ₘ-H.

10. Detergent and cleaning agent according to at least one of claims 1 to 9, **characterized in that** the compounds of formula (I) have the structure of formula (II) or the alkali metal or alkaline earth metal salts or partial neutralizates thereof
HO-C(CH₂-COOH)₂-COO-C₂H₄-OOC-COH-(CH₂COOH)₂ (II).

11. Detergent and cleaning agent according to at least one of claims 1 to 10, **characterized in that** the compounds of formula (I) are present as mixtures of a plurality of compounds of formula (I) which are liquid at 25°C.

12. Detergent and cleaning agent according to at least one of claims 1 to 11, **characterized in that** the compounds of the formula (I) have a radical of the formula (R¹OOC)ₐ-R²(OH)-COO- and a radical of the formula (R⁴OOC)_{b}-R³(OH)-COO-, these radicals having the same meaning.

13. Detergent and cleaning agent according to at least one of claims 1 to 12, **characterized in that** this is an agent for cleaning dishes.

14. Use of compounds of the formula (I)
(R¹OOC)ₐ-R²(OH)_{c}-COO-(CₙH₂ₙ-O)ₘ-OC-R³(OH)_{d}(COOR⁴)_{b} (I)
wherein R¹, R², R³, R⁴, a, b, c, d, n and m have the meaning defined in claim 1, as complexing agents in detergents and cleaning agents, in petroleum production or for water softening.

## Revendications

1. Agent de lavage et de nettoyage contenant des composés de formule (I)
(R¹OOC)ₐ-R²(OH)_{c-}COO-(CₙH₂ₙ-O)ₘ-OC-R³(OH)_{d}(COOR⁴)_{b} (I)
dans laquelle R¹ et R⁴ représentent, indépendamment l'un de l'autre, un atome d'hydrogène, un cation d'un métal, un cation ammonium, un groupe alkyle en C₁-C₆, un groupe cycloalkyle ayant trois à neuf atomes de carbone dans le cycle, un groupe aryle ayant cinq à dix atomes de carbone dans le cycle, un groupe aryle qui est substitué par un ou deux groupes alkyle, un groupe aryle qui est lié au groupe carboxyle par l'intermédiaire d'un groupe alkylène, -(CₙH₂ₙ-O)ₘ-H ou -O-R²(COOR¹)ₐ₊₁,
R² et R³ sont, indépendamment l'un de l'autre, des radicaux hydrocarbonés aliphatiques comportant de un à huit atomes de carbone,
a et b sont indépendamment des nombres entiers de 1 à 4,
c est un nombre entier de 0 à 4,
d est un nombre entier de 1 à 4,
n est égal à 2, 3 ou 4, et
m est égal à 1, 2, 3 ou 4, étant entendu que R¹ et R⁴ peuvent être différents au sein d'une même molécule dans le cadre des définitions données.

2. Agent de lavage et de nettoyage selon la revendication 1, **caractérisé en ce que** n est 2 ou 3, et m est 1 ou 2.

3. Agent de lavage et de nettoyage selon la revendication 2, **caractérisé en ce que** n est 2, et m est 1.

4. Agent de lavage et de nettoyage selon au moins l'une des revendications 1 à 3, **caractérisé en ce que** c et d valent 1.

5. Agent de lavage et de nettoyage selon au moins l'une des revendications 1 à 4, **caractérisé en ce que** R² représente un radical divalent de formule -CₒH₂ₒ- ou un radical trivalent de formule -CₚH₂ₚ₋₁< ou un radical tétravalent de formule >C_{q}H_{2q-2}<,
où o est un nombre entier de 2 à 4, de préférence de 2 à 3 et de manière particulièrement préférée de 2,
p est un nombre entier de 1 à 4, de préférence de 1 à 3 et plus préférablement 1 ou 2, et
q est un nombre entier de 2 à 4, de préférence de 2 à 3 et de manière particulièrement préférée de 3.

6. Agent de lavage et de nettoyage selon au moins l'une des revendications 1 à 5, **caractérisé en ce que** R² et R³ sont des restes qui dérivent de l'acide malique, de l'acide lactique, de l'acide tartronique, de l'acide tartrique, de l'acide isocitrique, de l'acide citrique, de l'acide acétylcitrique, de l'acide tartrique ou de l'acide mucilagineux après élimination des groupes carboxyle et des groupes hydroxyle.

7. Agent de lavage et de nettoyage selon la revendication 6, **caractérisé en ce que** R² et R³ sont des radicaux de formule (Ib), (Ic), (Id), (le), (If) ou (Ig)

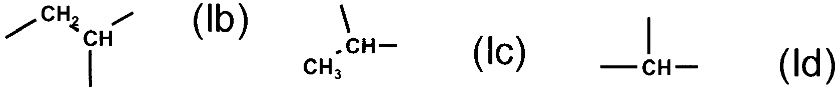

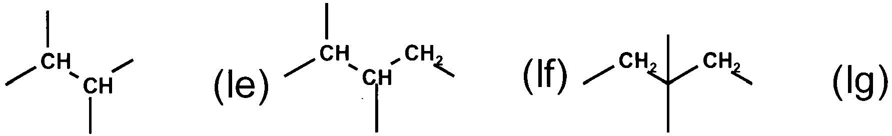

8. Agent de lavage et de nettoyage selon la revendication 6, **caractérisé en ce que** R² et R³ sont des radicaux qui dérivent de l'acide citrique après l'élimination des groupes carboxyle et du groupe hydroxyle.

9. Agent de lavage et de nettoyage selon au moins l'une des revendications 1 à 8, **caractérisé en ce que** a et b sont 2, **en ce que** R² et R³ sont des radicaux hydrocarbonés aliphatiques comportant trois atomes de carbone et **en ce que** R¹ et R⁴ sont des atomes d'hydrogène, des cations de métaux alcalins, des cations de métaux alcalino-terreux, des cations ammonium quaternaire ou des radicaux de formule -(CₙH₂ₙ-O)ₘ-H.

10. Agent de lavage et de nettoyage selon au moins l'une des revendications 1 à 9, **caractérisé en ce que** les composés de formule (I) présentent la structure de la formule (II) ou leurs sels alcalins ou alcalino-terreux ou leurs neutralisats partiels
HO-C(CH₂-COOH)₂-COO-C₂H₄-OOC-COH-(CH₂COOH)₂ (II).

11. Agent de lavage et de nettoyage selon au moins l'une des revendications 1 à 10, **caractérisé en ce que** les composés de formule (I) se présentent sous forme de mélanges de plusieurs composés de formule (I), qui sont liquides à 25°C.

12. Agent de lavage et de nettoyage selon au moins une des revendications 1 à 11, **caractérisé en ce que** les composés de formule (I) présentent un radical de formule (R¹OOC)ₐ-R²(OH)-COO- et un radical de formule (R⁴OOC)_{b}-R³(OH)-COO-, ces radicaux ayant la même signification.

13. Agent de lavage et de nettoyage selon au moins l'une des revendications 1 à 12, **caractérisé en ce que** celui-ci est un agent pour le nettoyage de la vaisselle.

14. Utilisation de composés de formule (I)
(R¹OOC)ₐ-R²(OH)_{c}-COO-(CₙH₂ₙ-O)ₘ-OC-R³(OH)_{d}(COOR⁴)_{b} (I)
dans laquelle R¹, R², R³, R⁴, a, b, c, d, n et m ont la signification définie dans la revendication 1, en tant qu'agent complexant dans des agents de lavage et de nettoyage, lors de l'extraction du pétrole ou pour l'adoucissement de l'eau.
